# EUROPEAN PATENT APPLICATION

(11) **EP 3 440 986 A1**
(43) Date of publication of application: **13.02.2019**
(21) Application number: 18188311.7
(22) Date of filing: 09.08.2018
(51) Int. Cl.: A61B 1/00, A61B 1/12, A61M 5/178

(54) **VOLUME EXPANDERS FOR ENDOSCOPES**

(30) Priority: 10.08.2017 US 201715674000
(71) Applicant: Ethicon, Inc., Somerville, NJ 08876 (US)
(72) Inventor: MORRISON, Todd, Irvine, CA 92618 (US)
(74) Representative: Small, Gary James

(57) **Abstract**

The disclosed subject matter is directed to volume expanders for use with endoscopes, particularly concerning low-pressure applications, such as sterilization. A volume expander may include an enclosure having a port and a wall including an outer surface and an inner surface, and a vent cap disposed proximate the port. The port may be a single port to which the vent cap is affixed. The volume expander permits gas within recesses of an endoscope to expand, but provides a barrier between the recesses of the endoscope and the surrounding environment.

## Description

### FIELD

The subject matter disclosed herein relates to sterilizing endoscopes in pressure chambers.

### BACKGROUND

Medical devices are typically sterilized before use in order to minimize the likelihood that a contaminated device might be used on a subject, which could cause an infection in the subject. Various sterilization techniques may be employed, such as steam, hydrogen peroxide, and vapor phase sterilization, either with or without a gas plasma and ethylene oxide (EtO). Each of these methods depends to a certain extent on the diffusion rates of the sterilization fluids, typically gases, upon the medical devices to be sterilized.

Certain sterilization techniques are conducted at pressures other than ambient pressure or atmospheric pressure. For example the STERRAD® System, STERRAD® NX System or STERRAD® 100NX System of Advanced Sterilization Products, Division of Ethicon US, LLC, a Johnson & Johnson company, are examples of sterilization systems that vaporize hydrogen peroxide and operate at low pressures, e.g., less than 200 millitorr. Such pressures present challenges for certain medical devices, such as endoscopes.

An endoscope is an elongate medical device that includes various tubular passages, some of which include components, e.g., wires. Typically, the tubular passages are contained within an elastomeric sleeve that is gas and liquid impermeable. Accordingly, a gas, such as air, is contained in the recesses outside the tubular passages and within elastomeric sleeve. This gas could cause the elastomeric sleeve to rupture during a low-pressure sterilization process if the pressure outside of the endoscope becomes lower than the pressure within the endoscope. In commercial endoscopes, rupturing may occur when the difference in pressure between the inside and the outside of the endoscope surpasses approximately 5 pounds per square inch. To avoid this eventuality, a vent cap may be connected to the endoscope to allow passage of gas into and out of the endoscope, i.e., into and out of the spaces between the tubular passages within the endoscope, such that the pressure inside and outside the endoscope remain substantially equal, including as the pressure is changed. For example, Olympus Corporation provides such a vent cap, part no. MB-156.

### SUMMARY

The disclosed subject matter is directed to volume expanders for use with endoscopes, particularly concerning low-pressure applications, such as sterilization. A volume expander may include an enclosure having a port and a wall including an outer surface and an inner surface, and a vent cap disposed proximate the port. The port may be a single port to which the vent cap is affixed. The enclosure may comprise an expandable material. The enclosure may be in a compact configuration The compact configuration may be a rolled up configuration.

In some embodiments, the enclosure of the volume expander may be a cylindrical tube having a distal end and a proximal end. The port may be disposed at the distal end of the tube. Further, a piston may be disposed within the tube. A backstop may be disposed on the proximal end of the tube. A plug may be disposed within the port. In some embodiments, the enclosure may be a syringe, the cylindrical tube a syringe tube, and the piston a syringe plunger. Further, the port may include a Luer fitting. A spring may also be disposed between the piston and backstop. The piston may be spherical.

In other embodiments, the enclosure may be composed of a rigid material, such as stainless steel. A gas, such as air, may be included within the enclosure. The pressure of the gas inside the enclosure may be a pressure less than atmospheric pressure, for example, between approximately 25 millitorr and 75 millitorr The enclosure may have a volume between approximately 10 ml and 100 ml. Further, a valve may be disposed within the port.

In some embodiments, the volume expander may include a neutralizing agent. The neutralizing agent may include, e.g., copper, palladium, and/or platinum.

In some embodiments, the volume expander may include a volume-expansion indicator. For example, in those embodiments where the enclosure is a balloon, the volume-expansion indicator may be a band encircling the balloon.

### BRIEF DESCRIPTION OF THE DRAWINGS

While the specification concludes with claims which particularly point out and distinctly claim the subject matter described herein, it is believed the subject matter will be better understood from the following description of certain examples taken in conjunction with the accompanying drawings, in which like reference numerals identify the same elements and in which:
Figure 1 shows a volume expander;
Figure 2 shows the volume expander of Figure 1 in a compact configuration;
Figure 3 shows an alternate embodiment of a volume expander; and
Figure 4 shows another alternate embodiment of a volume expander.

### DETAILED DESCRIPTION

The following description sets forth certain illustrative examples of the claimed subject matter. Other examples, features, aspects, embodiments, and advantages of the technology should become apparent to those skilled in the art from the following description. Accordingly, the drawings and descriptions should be regarded as illustrative in nature.

It should be understood that any of the examples and/or embodiments described herein may include various other features in addition to or in lieu of those described above. The teachings, expressions, embodiments, examples, etc. described herein should not be viewed in isolation relative to each other. Various suitable ways in which the teachings herein may be combined should be readily apparent to those of ordinary skill in the art in view of the teachings herein.

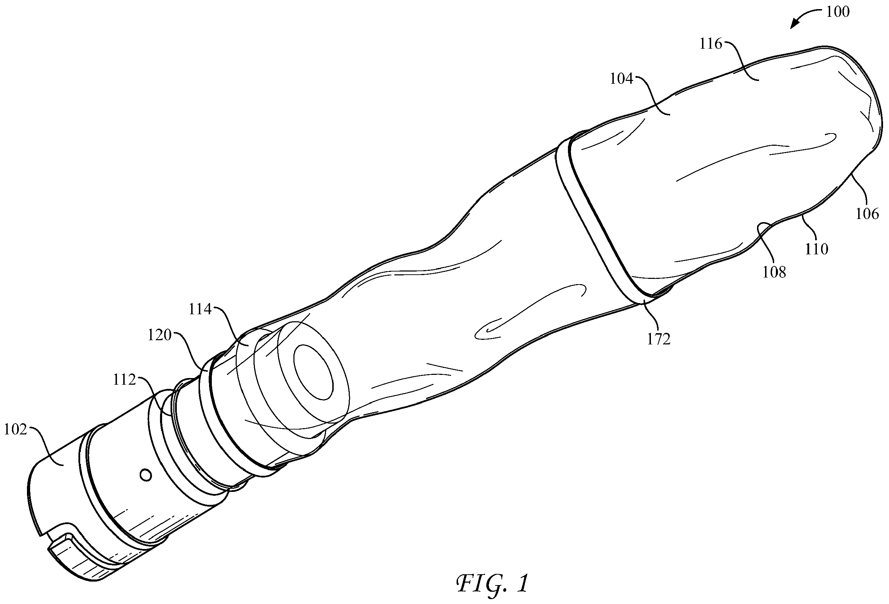

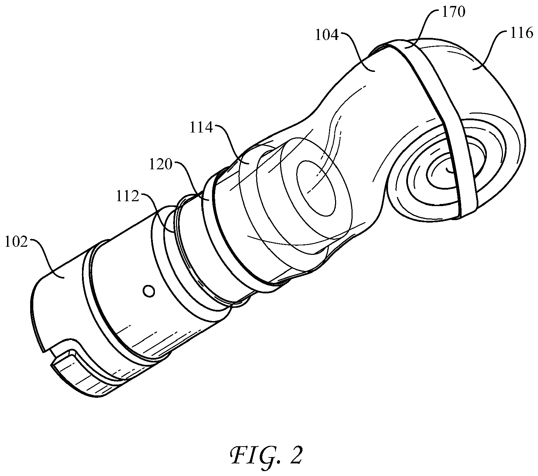

Figures 1 and 2 show a volume expander 100. Volume expander 100 includes various components and features, such as a vent cap 102. Volume expander 100 also includes an enclosure structure 104 comprising at least one wall 106 having an inner surface 108 and an outer surface 110. Enclosure structure 104 may also include a port 112, which in some embodiments may be disposed through a portion of wall 106. Vent cap 102 may be disposed proximate port 112. In some embodiments, vent cap 102 or a portion thereof, may be disposed within port 112. In some embodiments, vent cap 102 may be affixed to wall 106. In some embodiments, wall 106 includes a single port 112 and single vent cap 102. In some embodiments vent cap 102 may be a conventional vent cap or a commercially available vent cap such as Olympus vent cap, part no. MB-156. In some embodiments, conventional or commercially available vent caps may be modified to facilitate connection to enclosure structure 104. For example, tubing-connector barb 114 may be provided upon the surface of cap 102.

In various embodiments, enclosure structure 104 is expandable. For example, wall 106 may be fabricated from a resilient, elastomeric, or stretchable material, such as silicone rubber. Because of the elastomeric, stretchable nature of wall 106, the volume of enclosure structure 104 may increase when a pressure within volume expander 100 is greater than a pressure without because the pressure on inner surface 108 is greater than the pressure on outer surface 110.

In some embodiments, enclosure 104 may comprise a flexible and/or expandable material and have the form of a tube. For example, enclosure 104 may be an elastomeric tube that is sealed at one end. Alternatively, it may be a balloon, such as a tubular balloon 116. Balloon 116 may be connected to cap 102 by stretching a portion of the tubular balloon, typically port 112 and a portion of balloon 116 proximate thereto, over barb 114. In some embodiments, alternative or additional fixation devices or means may be employed, e.g., silicone glue or a collar 120. As seen in Figure 1, a portion of balloon 116 proximate to port 112 is affixed to cap 102 such that a portion of cap 102 is disposed through port 112 and inside balloon 116.

Tubular balloon 116 may be provided in a non-compact configuration (Figure 1) or a compact configuration (Figure 2). The compact configuration may include a rolled-up configuration or a folded configuration. Providing tubular balloon 116 in a compact configuration permits volume expander 100 to increase its volume by two mechanisms when subject to a pressure differential. That is, when the ambient pressure is less than the pressure within volume expander 100, balloon 116 may unroll (or unfold) to the non-compact configuration of Figure 1 and stretch to an expanded configuration, which has a volume greater than the non-compact configuration. Providing enclosure structure 104 in a compact configuration minimizes the number of gas (e.g., air) molecules contained within volume expander 100. This enables volume expander 100 to be used over a greater range of pressures than if it were provided in the non-compact configuration.

Volume expander 100 may further include devices or features that provide an indication of whether the volume of enclosure structure 104 changed during a sterilization procedure. Such a volume-expansion indicator may assist a health care work because during a sterilization procedure endoscopes are contained within a sterilization chamber, hidden from an operator's view. For example, a volume-expansion indicator may take the form of a first band 170 that may encircle balloon 116 in a compact configuration (Figure 2). Alternatively or additionally, the volume-expansion indicator may include a second band 172 that may encircle balloon 116 in a non-compact configuration (Figure 1). When a pressure differential is generated whereby pressure within balloon 116 is greater than outside balloon 116, balloon 116 transitions from the compact configuration to the non-compact configuration, thereby breaking or displacing band 170. As balloon 116 transitions from a non-compact configuration to an expanded configuration, band 172 may be broken or displaced. Thus, a health-care worker may inspect volume expander 100 following a sterilization procedure to determine that bands 170 and 172 have been broken or displaced. Alternatively, a volume expansion indicator may be a strain gauge. A strain gauge may be incorporated into volume expander 100 that may communicate to a user any changes in strain caused by balloon 116 changing configurations or expanding.

Volume expander 100 may further include a coupling to which a positive pressure air supply and pressure monitor could be connected. The air supply may be used to pressurize an endoscope to a safe pressure, and a pressure that will not disrupt balloon 116, e.g., by beginning a change of configuration from the compact configuration to the non-compact configuration. The pressure within the endoscope may then be monitored to perform a pressure decay test, which may provide an indication as to whether volume expander 100 is connected correctly to the endoscope and that volume expander 100 has no leaks. Alternatively, a negative pressure source may be connected to the coupling such that a pressure rise may be monitored for signs of leaks.

In typical usage, when a conventional vent cap is affixed to a vent port of an endoscope, fluid communication is established between the recesses of an endoscope (i.e., the spaces between the tubular passages of the endoscope) and the surrounding environment. When an endoscope having a vent cap affixed thereto is subject to a low-pressure sterilization procedure, such as a hydrogen-peroxide-sterilization procedure, peracetic-acid-sterilization procedure, or ozone-sterilization procedure, damage caused by a pressure differential inside and outside the endoscope may be avoided. However, sterilant may enter the recesses of the endoscope, which may damage the endoscope or reduce its biocompatibility. Endoscopes typically include lubricants in the recesses, such as molybdenum disulfide, which reacts with hydrogen peroxide to form sulfuric acid. The volume expanders described herein may reduce or eliminate damage to the endoscope caused by both pressure differentials and sterilants by providing gas within the endoscope extra room to expand while maintaining a barrier that prevents sterilant from entering the endoscope.

To further protect the endoscope from possible introduction of sterilants, in some embodiments, agents capable of neutralizing sterilant may be included within volume expander 100. A neutralizing agent may be provided within vent cap 102 such that any sterilant that passes through vent cap 102 becomes neutralized, thus minimizing or eliminating the damage that the sterilant might otherwise cause to an endoscope. Appropriate neutralizers may include, e.g., catalytic decomposing agents, such as copper, palladium, and platinum. Thus, if a sterilant becomes introduced into an endoscope during a sterilization procedure, e.g., by diffusing through a surface of the endoscope, vent cap 102 may assist in reducing damage to the endoscope caused by the sterilant. Conventional vent caps and modifications thereto, including incorporation of neutralizing agents, are described in U.S. Patent No. 5,634,880 to Feldman et al., U.S. Patent No. 5,807,238 to Feldman et al., and U.S. Patent No. 5,868,667 to Lin et al., each of which are incorporated by reference in their entirety herein.

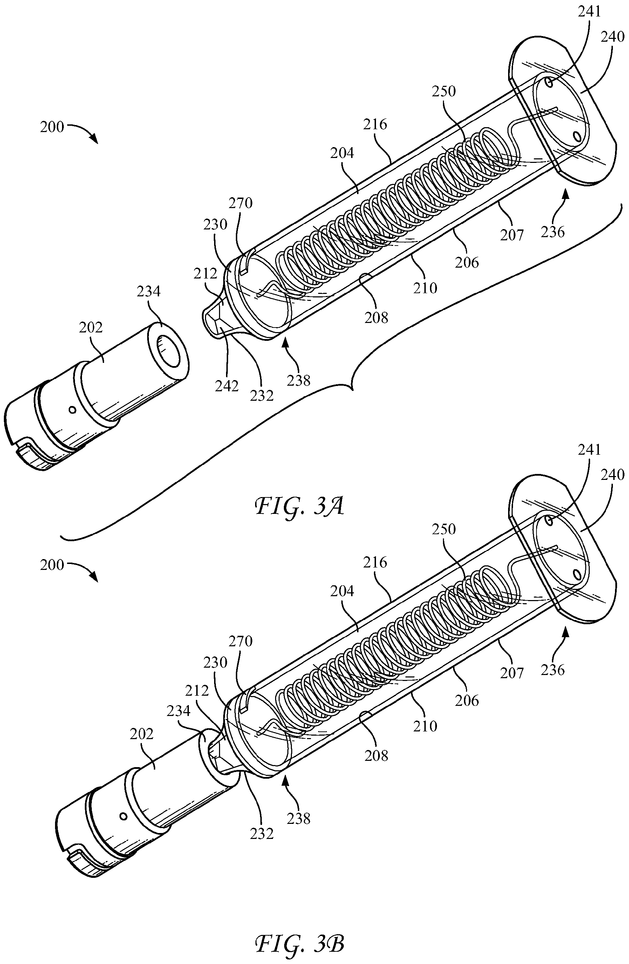

Figures 3A and 3B show an example of an alternative embodiment of a volume expander in accordance with the present disclosure. Volume expander 200 incorporates an enclosure structure 204 that is a syringe 216 having a proximal end 236 and a distal end 238, and comprising at least one wall 206, such as cylindrical wall 207, having an inner surface 208 and an outer surface 210. Syringe 216 also includes a port 212 disposed at distal end 238 and a piston (i.e., plunger) 230. A vent-cap 202 may be disposed proximate to port 212. In some embodiments, port 212 includes a first connector 232 and vent cap 202 includes a second connector 234. Connectors 232 and 234 may be connected to each other to join vent cap 202 to syringe port 212, such that any gas passing into or out of syringe 216 must pass through vent cap 202. In some embodiments, connectors 232 and 234 may be Luer-style fittings having a Luer taper, such as male or female Luer-lock or Luer-slip fittings.

In some embodiments, syringe 216 is provided with piston 230 disposed proximate to port 212, near to distal end 238, such that piston 230 may move toward the proximal end 236 when pressure outside the syringe becomes less than pressure inside the syringe. A backstop 240 may be disposed on proximal end 236 to prevent piston 230 from becoming ejected from syringe cylinder 207. Syringe backstops 240 are disclosed in U.S. Patent No. 5,667,495 to Bitdinger et al., which is hereby incorporated by reference in its entirety. In some embodiments, backstop 240 may include air vents 241 to accommodate air displaced by movement of piston 230.

In some embodiments, syringe 216 may be further provided with a plug 242 disposed within port 212. Plug 242 may have a volume substantially equal to the space defined by port 212 such that plug 242 and not gas is present therein. Accordingly, in those embodiments where port 212 comprises a male Luer-fitting, plug 242 may also have a Luer taper. Thus, when pressure outside syringe 216 becomes less than pressure inside the syringe causing piston 230 to become displaced, plug 242 may be forced into the volume defined by syringe cylinder 207.

In some embodiments, syringe piston 204 may further include a rod connected to the piston, as is common on conventional syringes. However, in some embodiments, syringe 202 lacks a rod because as pressure is lowered outside of the syringe causing piston 204 to move, a plunger rod may disturb or bump into other objects placed near to it. For example, if an endoscope to which volume expander 200 is connected is within a sterilization pack in a sterilization chamber, the sterilization pack may restrict movement of the plunger rod, and thus syringe piston 204, or it may cause the position of the endoscope within the pack to shift.

In some embodiments, syringe piston 230 may be a disc, e.g., a rubber disc, as is common in commercially available syringes. In other embodiments, piston 230 may have a spherical, conical, or cylindrical configuration. In some embodiments, movement of piston 230 may be restricted, e.g., by a spring 250 or a damping fluid having a high viscosity, to increase the force necessary to cause displacement of piston 230. Alternatively, vents 241 may not be included, or they may be plugged. In some embodiments, as reflected in Figure 3, spring 250 may be connected to backstop 240 and piston 230 such that spring 250 provides a resistive force against piston 230 for any movement of piston 230. However, in some embodiments, spring 250 may not be connected to piston 230 such that piston 230 may move freely until it encounters spring 250. Alternatively spring 250 may not be connected to backstop 240. Alternatively, spring 250 may not be connected to piston 230 or backstop 240.

In some embodiments volume expander 200 may include a volume-expansion indicator that provides an indication of whether the volume of enclosure structure 104 changed during a sterilization procedure. For example, the volume-expansion indicator may be an ink or marker 270 disposed on a surface of piston 230 that contacts inner surface 208 of wall 206. As piston 230 moves along wall 206, marker 270 marks inner surface of 208, providing an indication of the piston's movement.

Volume expanders 100 and 200 are exemplary embodiments of volume expanders having variable volumes. Other mechanisms for providing variable volumes include collapsible structures, such as a collapsible bellows or accordion bottles.

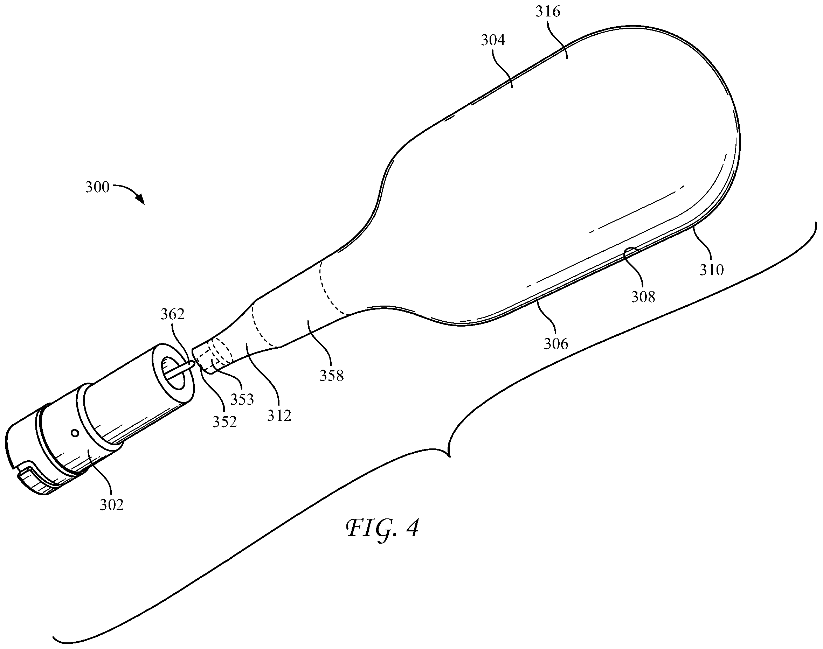

In some embodiments, a volume expander may have a fixed volume and include a sealed capsule containing a gas having a pressure lower than atmospheric pressure. Sealed capsules having a lower pressure within than without are known in the medical device field, e.g., the Vacutainer® manufactured by Becton, Dickinson and Company. As shown in Figure 4, volume expander 300 includes an enclosure structure 304, such as capsule 316, having at least one wall 306 including an inner surface 308 and an outer surface 310. Capsule 316 may be fabricated from a robust material, such as a strong plastic like polycarbonate or a corrosion resistant metal like stainless steel, having a thickness sufficient for resisting compression or expansion caused by a pressure differential between the inside and outside of capsule 316. Capsule 316 may further include a port 312 to which or within which a body 358 containing a neutralizing agent may be connected. In some embodiments, port 312 also includes a seal 352 disposed over its tip. In some embodiments, seal 352 may be an elastomeric membrane. In other embodiments, it may be a valve, such as a check valve, or valve capable of preventing flow in a single direction, such as a duck-bill valve. As shown in Figure 4, seal 352 is a duckbill valve 353. Thus, volume expander 300 may be provided in a fully sealed configuration and having a lower pressure within capsule 316 than ambient or atmospheric pressure. In some embodiments the pressure within capsule 316 may be between approximately 25 millitorr and 300 millitorr, for example, between approximately 25 millitorr and 75 millitorr, or approximately 50 millitorr. For these pressures, the volume of enclosure structure 304 should be substantially similar to or greater than the total volume of the recesses within the endoscope. For example, the volume of the enclosure structure 304 may between approximately 10 ml and 100 ml, or approximately 50 ml.

A vent cap 302 may also be provided that may be connected to port 312 and that includes a feature or device for unsealing seal 352, and hence, activating volume expander 300. For example, a needle may be included within cap 302 capable of puncturing a membrane seal. Alternatively, a finger 362 may be included that may push a check-valve or a duck-bill valve, e.g., valve 353, to an open configuration. Thus, when vent cap 302 is connected to port 312, seal 352 becomes unsealed. In various embodiments, vent cap 302 is attached to an endoscope's vent port before it is connected to port 312 of capsule 316 such that once capsule 316 is connected to port 312, the gas (likely air) within the recesses of the endoscope may be drawn into capsule 316, thereby reducing the amount of gas contained within the endoscope and providing additional volume for the gas to expand.

Volume expanders of the presently disclosed subject matter may be used to assist in sterilizing endoscopes according to the following exemplary methods. First, the endoscope should be prepared for sterilization. That is, it should at least be cleaned and dried, and optionally disinfected. Second, a volume expander, e.g., volume expander 100, 200, or 300, may be connected to a vent port of the endoscope. In the case of volume expander 300, vent cap 302 should be affixed to the vent port before connecting port 312 of capsule 316 to cap 302 to avoid premature opening of seal 352. After vent cap 302 is connected the vent port of the endoscope, port 312 of capsule 316 may be connected to vent cap 302, which causes finger 362 to open seal 352. Because the pressure within capsule 316 is less than the pressure within the endoscope, gas within the recesses of the endoscope move into capsule 316 to equalize the pressure therein with the pressure within the recesses of the endoscope. Third, the endoscope may be placed into a sterilization pack and positioned within a sterilization chamber of a sterilization system. Fourth, the sterilization system may be activated to commence a sterilization process. Fifth, during the sterilization process, pressure within the sterilization chamber is lowered, enlarging a pressure differential between the pressure in the chamber and the pressure inside the recesses of the endoscope not in fluid communication with the sterilization chamber. In the case of volume expander 100 including a rolled-up balloon 116, the pressure differential causes balloon 116 to unroll and expand. In the case of volume expander 200 including syringe 216, the pressure differential causes piston 230 to move from the distal end of the syringe toward the proximal end of the syringe. In the case of volume expander 300, although capsule 316 is rigid, capsule 316 is nonetheless able to accommodate gasses from within the endoscope and helps avoid rupturing of the endoscope because the number of molecules in the endoscope was reduced when seal 352 was opened, exposing the recesses of the endoscope to the lower pressure volume of capsule 316. Sixth, sterilant is introduced into the sterilization chamber. Should any sterilant enter the endoscope or volume expander, the neutralizing agent within the volume expander may assist in neutralizing it. Seventh, after sterilizing is complete, the sterilization chamber is vented or otherwise returned to atmospheric or near atmospheric pressure. In the case of volume expander 100, balloon 116 returns to the non-compact configuration. In the case of volume expander 200, piston 230 returns to its original position proximate the distal end of the syringe. Finally, a health-care worker may check to confirm that the volume-change indicators indicate that a volume change occurred. For example, in the case of volume expander 100, bands 170 and 172 should be displaced or broken. In the case of volume expander 200, a mark made by marker 270 should be visible along inner surface 210.

Having shown and described exemplary embodiments of the subject matter contained herein, further adaptations of the methods and systems described herein may be accomplished by appropriate modifications without departing from the scope of the claims. Some such modifications should be apparent to those skilled in the art. For instance, the examples, embodiments, geometries, materials, dimensions, ratios, steps, and the like discussed above are illustrative. Accordingly, the claims should not be limited to the specific details of structure and operation set forth in the written description and drawings.

## Claims

1. A volume expander, comprising;
an enclosure having a port and a wall including an outer surface and an inner surface, and;
a vent cap disposed proximate the port.

2. The volume expander of claim 1, wherein the port is a single port and the vent cap is affixed to the single port.

3. The volume expander of claim 1, wherein the enclosure comprises an expandable material.

4. The volume expander of claim 3, wherein the enclosure is in a compact configuration.

5. The volume expander of claim 4, wherein the compact configuration is a rolled-up configuration.

6. The volume expander of claim 1, wherein the enclosure includes a cylindrical tube having a distal end and a proximal end, wherein the port is disposed at the distal end, and further comprising a piston disposed within the cylindrical tube.

7. The volume expander of claim 6, further including a backstop disposed on the proximal end of the tube.

8. The volume expander of claim 6, further including a plug disposed within the port.

9. The volume expander of claim 7, wherein the enclosure is a syringe, the cylindrical tube is a syringe tube, the piston is a syringe plunger, and the port includes a Luer fitting.

10. The volume expander of claim 7, further comprising a spring connected to the piston and the backstop.

11. The volume expander of claim 10, wherein the piston is spherical.

12. The volume expander of claim 1, wherein the enclosure is composed of a rigid material.

13. The volume expander of claim 12, wherein the rigid material is stainless steel.

14. The volume expander of claim 12, further comprising a gas within the enclosure, the gas within the enclosure having a pressure less than atmospheric pressure.

15. The volume expander of claim 14, wherein the enclosure has a volume of between 10 ml and 100 ml and the gas within the enclosure has a pressure between approximately 25 millitorr and 75 millitorr.

16. The volume expander of claim 14, further comprising a valve disposed within the port.

17. The volume expander of claim 1, further comprising a neutralizing agent.

18. The volume expander of claim 17, wherein the neutralizing agent is selected from the group consisting of copper, palladium, and platinum.

19. The volume expander of claim 1, further comprising a volume-expansion indicator.

20. The volume expander of claim 19, wherein the enclosure is a balloon and the volume-expansion indicator is a band encircling the balloon.
